# EUROPEAN PATENT APPLICATION

(11) **EP 2 604 183 A1**
(43) Date of publication of application: **19.06.2013**
(21) Application number: 11193994.8
(22) Date of filing: 16.12.2011
(51) Int. Cl.: A61B 5/0478

(54) **Method and arrangement for detecting movement artifact from bioelectrical signals measured from the head of a patient**

(71) Applicant: General Electric Company, Schenectady, New York 12345 (US)
(72) Inventor: Särkelä, Mika, 02200 Espoo (FI); Virtanen, Juha, 00660 Helsinki (FI)
(74) Representative: Valkeiskangas, Tapio Lassi Paavali

(57) **Abstract**

The invention relates to a method and an arrangement for detecting movement artifact from bioelectrical signals measured from the head of a patient (1) comprising providing a base element (2) of flexible material, the base element comprising at least three electrodes (3) and a connector (4) connecting the at least three electrodes to a patient monitor (5), placing the base element on the patient's head so that the electrodes are in contact with the skin of the patient's head, and carrying out measuring of the bioelectrical signals. The method and the arrangement also comprise detecting tension of the base element (2) during the measuring of the bioelectrical signals by means of a tension sensing element (6).

## Description

### BACKGROUND OF THE INVENTION

The disclosure relates to method for detecting movement artifact from bioelectrical signals measured from the head of a patient, which method comprises providing a base element of flexible material, the base element comprising at least three electrodes and a connector connecting the at least three electrodes to a patient monitor, placing the base element on the patient's head so that the electrodes are in contact with the skin of the patient's head, and carrying out measuring of the bioelectrical signals. The disclosure relates also to an arrangement for measuring bioelectrical signals.

The brain waves of a human being provide an electrical signal at a very low level, e.g. a microvolt level. It is possible by connecting electrodes to the scalp of a person to detect and amplify said faint electrical signals. In the past those electrical signals were amplified and recorded on a paper strip chart showing an analog wavy line for each recording channel in an electroencephalograph (EEG). EEG has been used for many medical and testing purposes, for example testing of auditory, visual, somatosensory and motor systems and testing for pathological brain dysfunction. In the later years it has also been available EEG instruments which can simultaneously detect the faint signals and covert the signals to digital data for recording and analysis.

However, a major problem in obtaining an accurate recording of brain waves has been movement artifact which contains of electrical contaminants arising as a consequence of the subject's movements. Movement artifacts are caused by the relative movement of the electrode with respect to the scalp. This relative movement is very often caused by tension in the electrode wires or/and to a cap or net by which the electrodes have been connected to the head. The changes in contact of the electrode on the head result in changes in the impedance and induced potentials at the electrode-scalp interface, producing artifacts in the measured EEG signal. Also head movements can cause the electrode wires to generate a current as they move through the ambient 50/60 Hz magnetic field, producing electrode sway artifact.

In addition to movement artifacts, eye artifact is another source of electrical contaminants in EEG recordings. When the eyes move or the eyelids blink, electrical potentials can be recorded from electrodes located near the eyes. These measurements are electro-oculography (EOG) measurements. The eye related potential can also be picked up by EEG electrodes resulting in contaminated EEG signals. Sometimes movements and eye artifacts are present in the measured EEG signal simultaneously. In connection of motion artifacts all bioelectrical measurements measured from the head, i.e. also electromyography (EMG) measurements can be mentioned.

As discussed above movement artifacts for example in EEG signals are commonly recognized problem in automatic signal analysis. To some extent, movement artifacts can be identified based on the morphology or frequency content of the deflections in the signal, but use of independent additional information would be advantageous.

Use of acceleration transducers for measuring head motion is well-known in the field. It has been widely known for a person skilled in the art that if the acceleration transducer detects movements, the portion of the recording contaminated by such movement may be discarded or the portion of the recording contaminated may alternatively be appropriately corrected so that said contamination is eliminated. In this context, terms accelerometer and acceleration transducer relate to a device that measures a proper acceleration. An accelerometer thus measures weight per unit of a test mass, a quantity of acceleration known as g-force. In practice, commercially available accelerometers today are micro electromechanical systems consisting of proof mass and cantilever beam. Also, other types of accelerometers exist, such as piezoelectric accelerometers, for example. Piezoelectric accelerometers consist of spring-mass element that compresses piezoelectric material when the sprig-element stretches during the acceleration.

However at least three problems related to the use of accelerometers in connection with an EEG measurement system attached to the head of the patient can be identified.

One of the three problems discussed above is the cost. 3D-accelerometers cost about 1 EUR if used in very large quantities for example in connection with cell phones. For the quantities relevant to the sensors used in medical purposes, the estimated cost is few euros, which is significant when remembering that the EEG measurement systems are typically a single-use product.

The other problem is related to the supply of operating voltage to the accelerometer. Most commercially available acceleration transducers require electricity for the operation. Thus, EEG device should be equipped with output power supply connectors, and as a result, also the complexity of EEG measurement is increased as more leads are required to establish connection between the patient and the EEG device. The other alternative is to use accelerometers operating with batteries, however the use of batteries may significantly increase the weight, size and price of the system. Furthermore, during the measurement capacities of the batteries shall be monitored.

The third problem of the three problems discussed above relates to the movement of the electrode. It is commonly less recognized that the so-called movement artifact arises from the relative movement between skin and electrode. This means that if for example a light weight adhesive electrode is well attached to the forehead, head movement does not translate to movement artifact in EEG measurements simply because the electrode moves along with the head. A significant source of motion artifact is tugging of electrode lead wires, which does not manifest as head movement in the acceleration sensor. In case of the EEG measurement systems, such as electrode caps or nets, when the patient's head is moving in respect to the pillow, a significant tension is directed to some parts of the cap or net. This may lead to relatively large relative movement between electrodes and skin, even though the head movement in absolute measures was quite modest.

Owing to the shortcoming and disadvantage facts above it is easily understood that accelerometers are not so suitable devices to detect motion artifacts in EEG signal, i.e. in spite of the use of accelerometers there still remain practical problems to be solved.

### BRIEF DESCRIPTION OF THE INVENTION

The above-identified shortcomings, disadvantages and problems are addressed herein which will be understood by reading and understanding the following specification.

In an embodiment a method for detecting movement artifact from bioelectrical signals measured from the head of a patient comprising providing a base element of flexible material, the base element comprising at least three electrodes and a connector connecting the at least three electrodes to a patient monitor, placing the base element on the patient's head so that the electrodes are in contact with the skin of the patient's head, and carrying out measuring of the bioelectrical signals. The embodiment is characterized in that the method also comprising detecting tension of the base element during the measuring of the bioelectrical signals by means of a tension sensing element.

In another embodiment relating to an arrangement for detecting movement artifact from bioelectrical signals measured from the head of a patient the embodiment is characterized in that the base element is provided with a tension sensing element detecting tension of the base element.

The benefit of the embodiments above is sensitivity to the most relevant phenomenon concerning movement artifact, i.e. tension on the base element, i.e. for example on the net or cap into which the electrodes have been attached, not to head movement as such. Referring to the benefits of the embodiments described above it can be mentioned here that tension sensing elements such as piezoelectric sensors are also inexpensive when compared to for example accelerometers, furthermore piezoelectric sensors do not require operating voltage, contrary to accelerometers. As a further example of the benefits of the embodiments it can be mentioned also that the electronics needed with piezoelectric sensors can be materialized in an, inexpensive way with only two lead wires.

### BRIEF DESCRIPTION OF THE DRAWINGS

Figures 1 and 2 show a basic principle of the first embodiment seen in two directions,
Figure 3 shows principally the second embodiment,
Figure 4 shows principally the third embodiment,
Figure 5 shows a structural detail of the embodiments shown in figures 1 - 4, and
Figure 6 shows another structural detail of the embodiments shown in Figures 1 - 4.

### DETAILED DESCRIPTION OF THE INVENTION

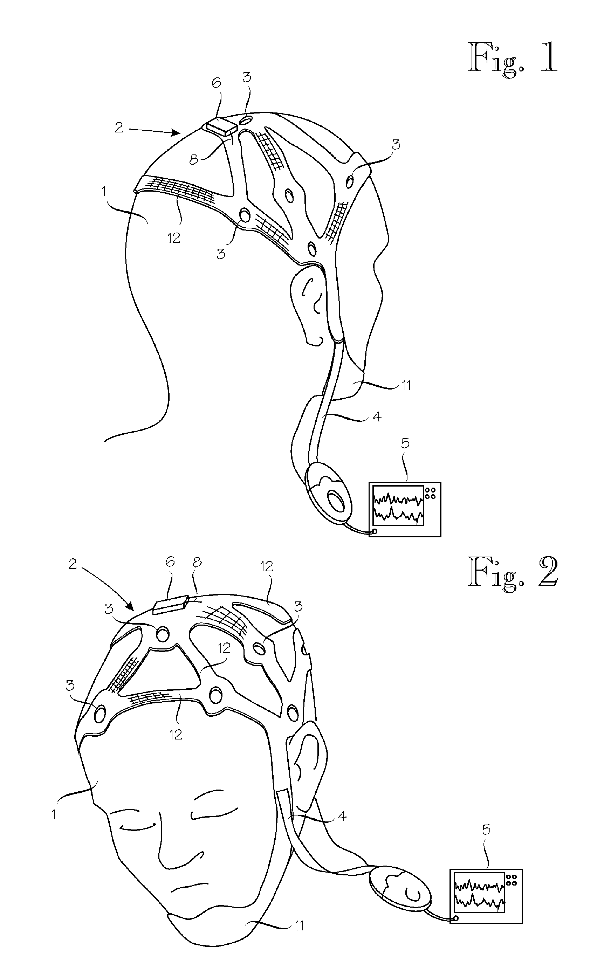

Figures 1 and 2 show principally the first embodiment. Reference number 1 shows a head of the patient to be investigated. Reference number 2 shows a base element. The base element may be for example a hat or a cap covering the upper portion of the head. In the embodiment of figures 1 and 2 the base element is formed as construction using flexible straps or stripes 12 connected to each other to for a netlike structure. It is however quite possible to form the base element of figures 1 and 2 by using a flexible net structure or another appropriate structure etc. In the embodiment of figures 1 and 2 the base element comprises also a strap part 11 extending under the chin of the patient. This is not however the only possibility to fasten the base element onto the head of the patient but the base element can be fastened to the head also without the part extending under the chin.

Reference number 3 shows electrodes for measuring bioelectrical signal from the head of the patient.

Reference number 4 shows a connector connecting the electrodes to a patient monitor 5. Although wired connection is presented in figures 1 and 2, also wireless connection can be used.

As described earlier the point in the embodiment described in figures 1 and 2 is the use of tension sensing elements. The tension sensing elements may be piezoelectric sensors or other appropriate elements. Piezoelectric sensors referred to above are strips or film elements, which generate large voltage difference between the sides under the strain, such as bending or stretching. These sensors are typically very sensitive. The dependence between the voltage and the bending force or distance is highly non-linear.

As also described earlier tension stresses on the base element create motion artifacts. In the embodiments described above the tension sensing element 6, for example piezoelectric sensor 6 is used to detect said tension stresses in the base element, and therefore the base element 2 is provided with said tension sensing element 6, and tension of the base element 2 is detected by using the tension sensing element 6 such as piezoelectric sensor described above.

The core of the embodiment shown in figures 1 and 2 is that tension sensing element 6 is provided into the base element 2. This tension sensing element 6 such as piezoelectric sensor is a good indicator of tension changes in the base element 2.

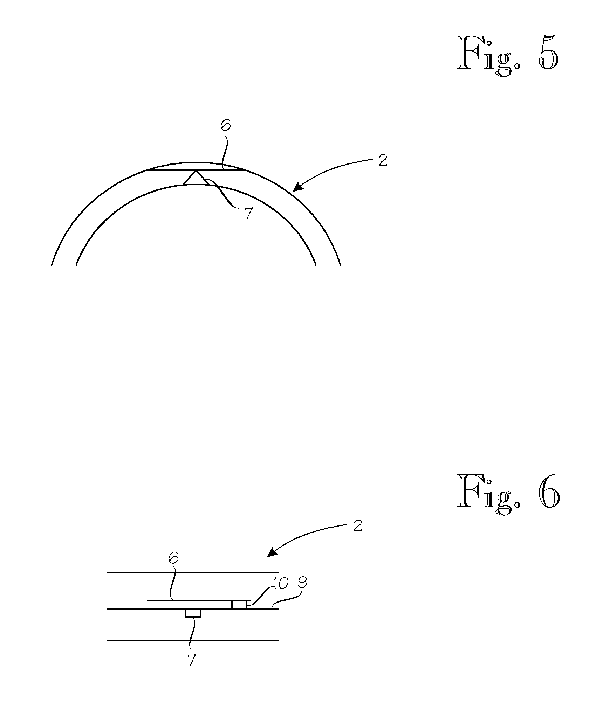

Referring further to the tension sensing element 6 such as piezoelectric sensor it is preferable that the tension sensing element 6 is a pre-stressed sensor. Flexible strap 12 pulling the tension sensing element 6 such as piezoelectric sensor to conform to the head circumference is a practical and good way to materialize said pre-stressing. However it has been found advantageous to place a specifically designed support element 7 under the tension sensing element 6. This detail is described in figure 5. The goal is to focus the counter-force from the head to the center of the tension sensing element 6 such as piezoelectric sensor to maximize the bending effect from the flexible strap 12 of the base element 2.

The tension sensing element 6 can be placed at any appropriate location in the base element but it has been found that in the embodiment of figures 1 and 2 the tension sensing element it is preferable that the tension sensing element is placed on the top of head as shown in figures 1 and 2, i.e. so that the tension sensing element 6 such as piezoelectric sensor is placed on the top of head when the measuring step is carried out. The tension sensing element 6 can be placed above an electrode 3 sensor or alternatively close to an electrode 3 according to the existing need etc.

The tension sensing element 6 such as piezoelectric sensor can be connected electrically to the monitor by using separate leads or conductors 8. This is however not the only possibility but it is also possible to bond the tension sensing element 6 from one end to the conducting elements 9 used to connect the electrodes 3. The conducting elements 9 are shown schematically in figure 6.

Figures 1 and 2 show the embodiment only principally, i.e. showing the location of the tension sensing element in the base element 2. It is however very advantageous to form the base element 2 by using tube-like straps so that the tension sensing element is embedded into the base element. Figure 5 shows said basic principle. Tube-like straps of the base element 2 may be made of for example of textile material. The cap or hat part covering the top of the head may be manufactured by using the straps described above.

Figure 6 shows principally one way to materialize the construction shown in figures 1, 2 and 5. It must be noted here that figures 5 and 6 are purely schematical, i.e. said figures show only the principle, and therefore different details are shown in a way separately to clarify the basic idea. The conducting elements 9 connecting for example the electrodes 3 run inside the tube-like strap of the base element 2. The tension sensing element 6 such as piezoelectric sensor is bonded by using bonding 10 to the conducting elements 9. Support element 7 is placed under the center part of the tension sensing element 6 in to create the effect shown basically in figure 5. The shape of the support element 7 is not critical, for example a triangular or conical shape can be used so that the force is focused optimally to the tension sensing element, for example to the center of the tension sensing element, and so that the support element is comfortable for the underlying skin.

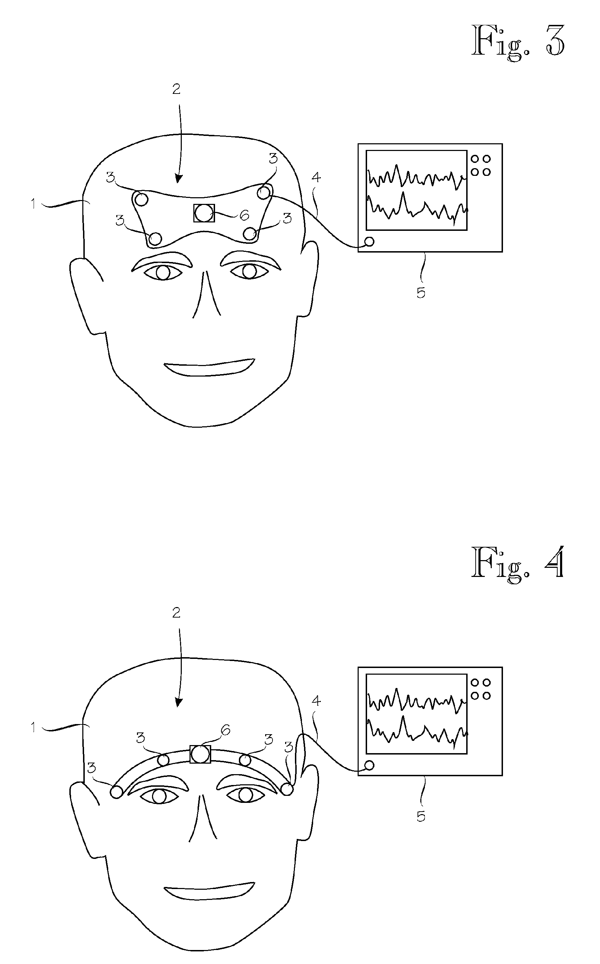

Figures 3 and 4 show another embodiment. In figures 3 and 4 the same reference numbers are used is corresponding purposes as in figures 1, 2, 5 and 6. In this embodiment the base element 2 is placed on the forehead of the patient 1. Figures 3 and 4 show principally location of the tension sensing element 6 such as piezoelectric sensor. The construction shown in Figures 3 and 4 can be materialized basically in the way as shown in figures 5 and 6, i.e. the tension sensing element can me embedded into the base element 2 and support element 7 can be used in the same way as described in figures 5 and 6. The base element 2 of the embodiments shown in figures 3 and 4 can be placed onto the forehead of the patient in any appropriate way, i.e. by using straps or adhesive elements etc.

This written description uses examples to disclose the invention, including the best mode, and also to enable any person skilled in the art to practice the invention, including making using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. Method for detecting movement artifact from bioelectrical signals measured from the head of a patient (1) comprising: providing a base element (2) of flexible material, the base element comprising at least three electrodes (3) and a connector (4) connecting the at least three electrodes to a patient monitor (5), placing the base element on the patient's head so that the electrodes are in contact with the skin of the patient's head, and carrying out measuring of the bioelectrical signals, **characterized in that** the method also comprising detecting tension of the base element (2) during the measuring of the bioelectrical signals by means of a tension sensing element (6).

2. The method of claim 1, further comprising pre-stressing the tension sensing element (6) by applying tension force to the base element (2) during the placement of the base element (2) on the patient's head.

3. The method of claim 2, **characterized in that** the tension force is applied by conforming the base element (2) onto the form of the patient's head.

4. The method of claim 1, 2 or 3, **characterized in** the tension sensing element (6) is pre-stressed by placing a support element (7) under the tension sensing element (6), the support element (7) focusing the counter force from the head (1) to the center of the tension sensing element (6).

5. The method of the claims 1, 2 or 3, **characterized in that** the tension sensing element (6) is placed so that it is positioned on the top of head (1) during the measuring of the bioelectrical signals.

6. Arrangement for detecting movement artifact from bioelectrical signals measured from the head of a patient (1), the arrangement comprising a base element (2) of flexible material, the base element comprising at least three electrodes (3) and a connector (4) connecting the at least three electrodes to a patient monitor (5), the electrodes being configured to be in contact with the skin of the patient's head when the base element is placed onto the head of the patient, **characterized in that** the base element (2) is provided with a tension sensing element (6) detecting tension of the base element (2).

7. Arrangement of claim 6, **characterized in that** the tension sensing element (6) is a pre-stressed tension sensing element.

8. Arrangement of claim 7, **characterized in that** pre-stressing is materialized by applying tension force to the base element (2) during the placement of the base element on the patient's head.

9. Arrangement of claim 7 or 8, **characterized in that** pre-stressing is materialized by providing a support element (7) under the tension sensing element (6), the support element (7) focusing the counter force from the head (1) to the center of the tension sensing element (6).

10. Arrangement of claim 6, 7, 8 or 9, **characterized in that** the base element (2) is configured so that when placed onto the head of the patient (1), at least some of the electrodes (3) are in contact with the top of the head or with the forehead of the head.

11. Arrangement of claim 6, 7, 8 or 9, **characterized in that** the tension sensing element (6) is arranged to the base element (2) so that it is positioned on the top of the head when the base element is placed onto the head of the patient, and **in that** the arrangement is provided with a strap part (11) extending under the chin of the patient.

12. Arrangement of any of the claims 6 - 11, **characterized in that** the tension sensing element (6) is placed above one of the electrodes (3).

13. Arrangement of any of the claims 6 - 12, **characterized in that** the tension sensing element (6) is embedded into the base element (2).

14. Arrangement of claim 13, **characterized in that** the tension sensing element (6) is embedded between the material layers of the base element (2).

15. Arrangement of any of the claims 6 - 14, **characterized in that** the bioelectrical signals are electroencephalography (EEG), electromyography (EMG) or electrooculography (EOG) signals.

## Amended claims

### Amended claims in accordance with Rule 137(2) EPC.

**1.** Method for detecting movement artifact from bioelectrical signals measured from the head of a patient (1) comprising: providing a base element (2) of flexible material, the base element comprising at least three electrodes (3) and a connector (4) connecting the at least three electrodes to a patient monitor (5), placing the base element on the patient's head so that the electrodes are in contact with the skin of the patient's head, and carrying out measuring of the bioelectrical signals, **characterized in that** the method further comprises providing a tension sensing element (6) for detecting the movement artifact, and measuring tension of the base element (2) with the tension element (6) during the measuring of the bioelectrical signals.

**2.** The method of claim 1 further comprising pre-stressing the tension sensing element (6) by applying tension force to the base element (2) during the placement of the base element (2) on the patient's head.

**3.** The method of claim 2, **characterized in that** the tension force is applied by conforming the base element (2) onto the form of the patient's head.

**4.** The method of claim 1, 2 or 3, **characterized in that** the tension sensing element (6) is pre-stressed by placing a support element (7) under the tension sensing element (6), the support element (7) focusing the counter force from the head (1) to the center of the tension sensing element (6)

**5.** The method of the claims 1,2 or 3, **characterized in that** the tension sensing element (6) is placed so that it is positioned on the top of head (1) during the measuring of the bioelectrical signals.

**6.** Arrangement for detecting movement artifact from bioelectrical signals measured from the head of a patient (1), the arrangement comprising a base element (2) of flexible material, the base element comprising at least three electrodes (3) and a connector (4) connecting the at least three electrodes to a patient monitor (5), the electrodes being configured to be in contact with the skin of the patient's head when the base element is placed onto the head of the patient, **characterized in that** the base element (2) is provided with a tension sensing element (6) for detecting the movement artifact which tension sensing element is configured to measure tension of the base element (2) during the measuring of the bioelectrical signals.

**7.** Arrangement of claim 6, **characterized in that** the tension sensing element (6) is a pre-stressed tension sensing element.

**8.** Arrangement of claim 7, **characterized in that** pre-stressing is materialized by applying tension force to the base element (2) during the placement of the base element on the patient's head.

**9.** Arrangement of claim 7 or 8, **characterized in that** pre-stressing is materialized by providing a support element (7) under the tension sensing element (6), the support element (7) focusing the counter force from the head (1) to the center of the tension sensing element (6).

**10.** Arrangement of claim 6, 7, 8 or 9, **characterized in that** the base element (2) is configured so that when placed onto the head of the patient (1), at least some of the electrodes (3) are in contact with the top of the head or with the forehead of the head.

**11.** Arrangement of claim 6, 7, 8 or 9, **characterzed** in that the tension sensing element (6) is arranged to the base element (2) so that it is positioned on the top of the head when the base element is placed onto the head of the patient, and in that the arrangement is provided with a strap part (11) extending under the chin of the patient.

**12.** Arrangement of any of the claims 6-11, **characterized in that** the tension sensing element (6) is placed above one of the electrodes (3).

**13.** Arrangement of any of the claims 6 - 12, **characterized in that** the tension sensing element (6) is embedded into the base element (2).

**14.** Arrangement of claim 13, **characterized in that** the tension sensing element (6) is embedded between the material layers of the base element (2).

**15.** Arrangement of any of the claims 6 - 14, **characterized in that** the bioelectrical signals are electroencephalography (EEG), electromyography (EMG) or electrooculography (EOG) signals.
